# EUROPEAN PATENT SPECIFICATION

(11) **EP 3 355 952 B1**
(45) Date of publication and mention of the grant of the patent: **01.07.2020**
(21) Application number: 16805659.6
(22) Date of filing: 17.11.2016
(51) Int. Cl.: G16H 20/17, G16H 20/40, A61M 1/28, A61M 3/02

(54) **MEDICAL FLUID PUMPING SYSTEMS AND RELATED METHODS**
MEDIZINISCHE FLÜSSIGKEITSPUMPSYSTEME UND ZUGEHÖRIGE VERFAHREN
SYSTÈMES DE POMPAGE DE FLUIDE MÉDICAL ET PROCÉDÉS ASSOCIÉS

(30) Priority: 04.12.2015 US 201514959678
(43) Date of publication of application: 08.08.2018
(73) Proprietor: Fresenius Medical Care Holdings, Inc., Waltham, MA 02451-1457 (US)
(72) Inventor: EGLEY, Bert D., Walnut Creek California 94598 (US)
(74) Representative: Conroy, John
(86) International application number: PCT/US2016/062428
(87) International publication number: WO 2017/095636

(56) References cited:
- WO-A1-99/06082
- WO-A2-2013/067359
- US-B1- 6 290 669

## Description

### TECHNICAL FIELD

This disclosure relates generally to medical fluid pumping systems and methods of implementing such systems and related devices to reduce infections.

### BACKGROUND

Dialysis is a treatment used to support a patient with insufficient renal function. The two principal dialysis methods are hemodialysis and peritoneal dialysis.

During hemodialysis ("HD"), the patient's blood is passed through a dialyzer of a dialysis machine while also passing a dialysis solution or dialysate through the dialyzer. A permeable membrane in the dialyzer separates the blood from the dialysate within the dialyzer and allows diffusion and osmosis exchanges to take place between the dialysate and the blood stream. These exchanges across the membrane result in the removal of waste products, including solutes like urea and creatinine, from the blood. These exchanges also regulate the levels of other substances, such as sodium and water, in the blood. In this way, the dialysis machine acts as an artificial kidney for cleansing the blood.

During peritoneal dialysis ("PD"), a patient's peritoneal cavity is periodically infused with sterile aqueous solution, referred to as PD solution or dialysate. The membranous lining of the patient's peritoneum acts as a natural permeable membrane that allows diffusion and osmosis exchanges to take place between the solution and the blood stream. These exchanges across the patient's peritoneum result in the removal of waste products, including solutes like urea and creatinine, from the blood, and regulate the levels of other substances, such as sodium and water, in the blood.

Many PD machines are designed to automatically infuse, dwell, and drain dialysate to and from the patient's peritoneal cavity. The treatment typically lasts for several hours, often beginning with an initial drain cycle to empty the peritoneal cavity of used or spent dialysate. The sequence then proceeds through the succession of fill, dwell, and drain phases that follow one after the other. Each phase is called a cycle.

Patients undergoing PD are susceptible to infections, such as peritonitis. Infections such as peritonitis can be caused by the presence of bacterial biofilms on PD catheters or bacteria in the peritoneal cavity. The bacteria may be introduced via the tubing, connectors, or other components of the PD machine. The bacteria may be introduced into a patient cavity, such as the peritoneum, during dialysis and may cause, vomiting, abdominal tenderness and other symptoms or infections, which in some cases can be fatal.

Irrigation of infected cavities may be used to remove bacteria and treat infections such as peritonitis. Common irrigation methods include rinsing and flushing the cavity with saline or anti-bacterial solutions. Some irrigation devices include bags and clamps with gravity fed fluid passively introduced at room temperature while others include irrigation devices where gravity fed fluid passes through automated flow control devices.

WO 2013/067359 describes a peritoneal dialysis system that includes a control system that can adjust the volume of fluid infused into the peritoneal cavity to prevent the intraperitoneal fluid volume from exceeding a predetermined amount. The control system can adjust by adding one or more therapy cycles, allowing for fill volumes during each cycle to be reduced.

US 6,290,669 describes a peritoneal dialysis apparatus that includes a holder device for holding a container of a dialysis fluid to be introduced into the peritoneal chamber of a subject; a catheter device insertable through the abdominal wall of the subject for introducing the dialysis fluid into the peritoneal chamber for draining the dialysis fluid therefrom; and a conduit device fluidly connecting a container held by the holder device to the catheter device.

WO 99/06082 describes an automated peritoneal dialysis machine is provided which is capable of selecting and changing the composition of dialysate delivered to a patient (10) in the course of treatment.

### SUMMARY

The invention is defined in the claims.

The invention provides systems and methods for irrigation of infected cavities, including pulsing the fluid pressure in the cavity so that the irrigation fluid can reach and mix with stagnant pockets of infection , pulsing the fluid pressure in the catheter so that bacterial biofilms can be removed and drained, using an optical detector to measure a characteristic of the effluent (such as cloudiness or white blood cell count) and repeating a series of fill - dwell & pulse - drain cycles until the effluent characteristic is below a certain level, and by heating the irrigation fluid.

In one example, a medical fluid pumping system includes a medical fluid pump chamber. A fluid exchange conduit is fluidly coupled to the medical fluid pump chamber. At least one pump configured to pump medical fluid to and drain medical fluid from a cavity of a patient via a patient line is coupled to the fluid exchange conduit. A pump controller is communicably coupled to the at least one pump. The pump controller is configured to cause the pump to transmit fluid pulses.
In particular implementations, the at least one pump is configured to transmit fluid pulses through the patient line. The at least one pump may be configured to pump dialysate to and drain dialysate from a peritoneal cavity of the patient via the patient line coupled to the fluid exchange conduit. The pump controller is configured to cause the pump to transmit fluid pulses during a dwell cycle, in accordance with particular implementations. The pump controller may be configured to cause the pulses to occur at a rate of 1Hz or less. In particular implementations, the pump controller is configured to cause the pulses to occur at a rate in the range of 100Hz to 500Hz. The pump controller is configured to cause the pulses to occur sinusoidally, in accordance with certain implementations. The patient line may include a catheter. The pump controller is configured to cause the pulses to occur at a resonant frequency of the catheter, in particular implementations. The pump controller is configured to cause the at least one pump to pump dialysate or irrigation fluid into the peritoneal cavity before causing the at least one pump to transmit fluid pulses into the peritoneal cavity during a dwell cycle, in particular implementations. The pump controller may be configured to cause the at least one pump to drain fluid from the peritoneal cavity via the patient line after causing the at least one pump to transmit fluid pulses to the fluid compartment during the dwell cycle. In particular implementations, the medical fluid pump chamber is defined by a disposable cassette. The medical fluid pumping system may include an optical sensor coupled to at least one of the fluid exchange conduit and the medical fluid pump chamber, the optical sensor communicably coupled to the pump controller. In particular implementations, the pump controller is configured to cause the at least one pump to transmit fluid pulses in response to a reading of the optical sensor being above or below a predetermined threshold. The medical fluid pumping system includes a first container and a second container in fluid communication with the fluid exchange conduit, the first container containing the dialysate, the second container containing an irrigation fluid, in certain implementations. The medical fluid pumping system may include of claim a heater coupled to at least one of the first disposable unit and the second disposable unit. The at least one pump may include a piston pump. The at least one pump may include a piezoelectric pump. The cavity may include a urinary track bladder.

In one example a medical fluid pumping system includes a medical fluid pump chamber. A fluid exchange conduit is fluidly coupled to the medical fluid pump chamber. The medical fluid pumping system includes at least one pump configured to pump medical fluid to and drain medical fluid from a cavity of a patient via a patient line coupled to the fluid exchange conduit. The medical fluid pumping system includes a rotary insert positioned in the fluid exchange conduit. An actuator is coupled to the rotary insert. A flow controller is electrically coupled to the actuator to cause the rotary insert to rotate in concert with actuation of the at least one pump to transmit fluid pulses to the medical fluid in the cavity during a dwell cycle.

In particular implementations, the at least one pump is configured to pump dialysate to and drain dialysate from a peritoneal cavity of the patient via the patient line coupled to the fluid exchange conduit. The flow controller may be configured to cause the pulses to occur at a rate in the range of 100Hz to 500Hz. The flow controller is configured to cause the pulses to occur sinusoidally, in particular embodiments. The flow controller is configured to cause the pulses to occur at a resonant frequency of the patient line, in particular embodiments. The medical fluid pump chamber may be defined by a disposable cassette. In particular embodiments, the medical fluid pumping system includes an optical sensor coupled to at least one of the fluid exchange conduit and the medical fluid pump chamber, the optical sensor communicably coupled to the flow controller. The flow controller may be configured to cause transmission of fluid pulses in response to a reading of the optical sensor being above or below a predetermined threshold.

One method includes during a dwell cycle of a peritoneal dialysis treatment, generating fluid pulses in a patient line connected to a patient cavity of a patient by pumping dialysate or irrigation fluid through the patient line and into the patient cavity. Generating fluid pulses includes pumping dialysate or irrigation fluid from a medical fluid pump chamber via a pump through an outlet in the medical fluid pump chamber configured for coupling to the patient line.

In particular embodiments, generating fluid pulses includes pulsing the flow rate of the pump. Generating fluid pulses may include moving a valve positioned in one of the fluid compartment and the patient line, in accordance with particular embodiments. Generating fluid pulses may include pulsing the dialysate or irrigation fluid at a frequency in the range of 100Hz to 500Hz. In particular embodiments, generating fluid pulses includes pulsing the dialysate or irrigation fluid at a frequency corresponding to a resonant frequency of the patient line. The method includes irrigating a peritoneal cavity with an irrigation fluid in an irrigation compartment fluidly coupled to the patient line. The method may include measuring a level corresponding to a characteristic of the effluent of the dialysate or irrigation fluid. In particular embodiments, the method includes filling the peritoneal cavity with an irrigation fluid and pumping, pulsing, dwelling, and draining the dialysate or irrigation fluid cyclically until the level indicates that the characteristic of the effluent of the dialysate or irrigation fluid is below a predetermined threshold. The method may include elevating the temperature of the irrigation fluid above. 37 degrees Celsius (98.6 degrees Fahrenheit).

One method includes pumping, via a pump, a medical fluid from a medical fluid pump chamber to a cavity of a patient via a patient line connected to the medical fluid chamber and pulsing the medical fluid through the patient line.

In particular embodiments, the cavity includes at least one of a urinary tract and a bladder.

Implementations can include one or more of the following advantages. In some implementations, devices related to treatment of an internal cavity with medical fluid can advantageously be mechanically treated via pulsed fluids despite changes in resistance of bacteria to treatment fluids. Some of the treatment methods described herein offer additional advantages related to the ability to implement the treatment methods during peritoneal dialysis cycles. Certain treatment methods described herein also advantageously allow localized and/or targeted treatment of bacterial biofilms on a catheter and in a cavity for both prevention and treatment of infections.

Other aspects, features, and advantages will be apparent from the description and drawings, and from the claims.

### DESCRIPTION OF DRAWINGS

FIG. 1A is a perspective view of a peritoneal dialysis ("PD") system that includes a PD cycler attached to a patient.
FIG. 1B is a perspective view of a peritoneal dialysis ("PD") system that includes a PD cycler configured for attachment to a patient.
FIG. 2A is a perspective view of the PD cycler and a PD cassette of the PD system of FIG. 1.
FIGS. 2B and 2C are schematics of operation of piston pumps of the PD cycler of FIG. 2A.
FIG. 3 is a flow diagram demonstrating operation of a PD system for pulsing.
FIGS. 4A-4F show a finite element analysis of resonant frequencies of a catheter that can be used with the PD cycler of FIG. 1 for PD treatments.
FIG. 5 is a flow diagram demonstrating operation of a PD system for pulsing responsive to an optical sensor system.
FIG. 6A is a flow diagram demonstrating operation of a PD system for pulsing at prescribed resonant frequencies.
FIG. 6B is a flow diagram demonstrating operation of a PD system for pulsing at identified resonant frequencies.

### DETAILED DESCRIPTION

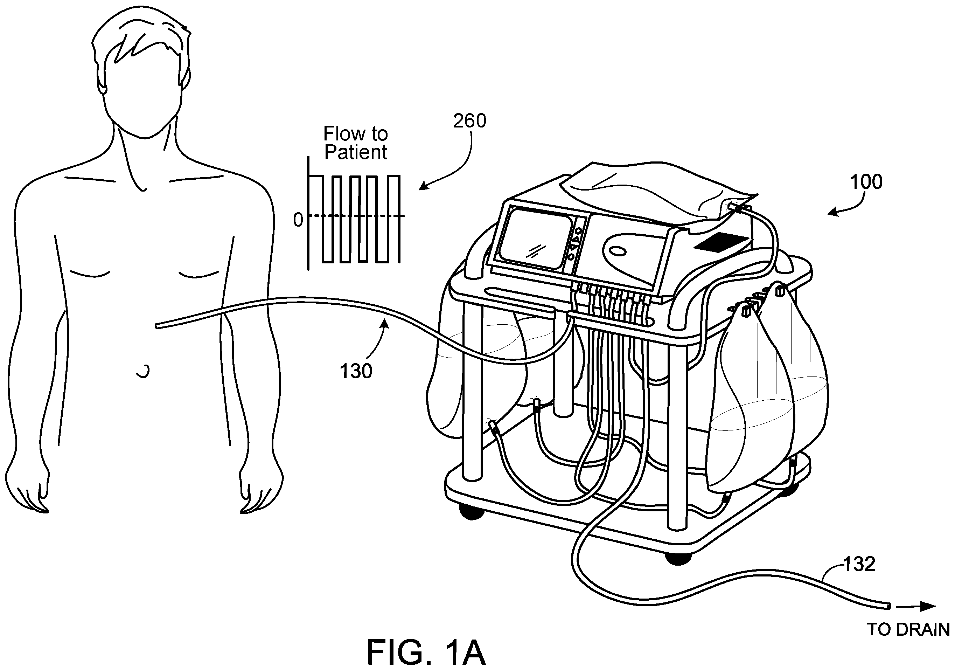

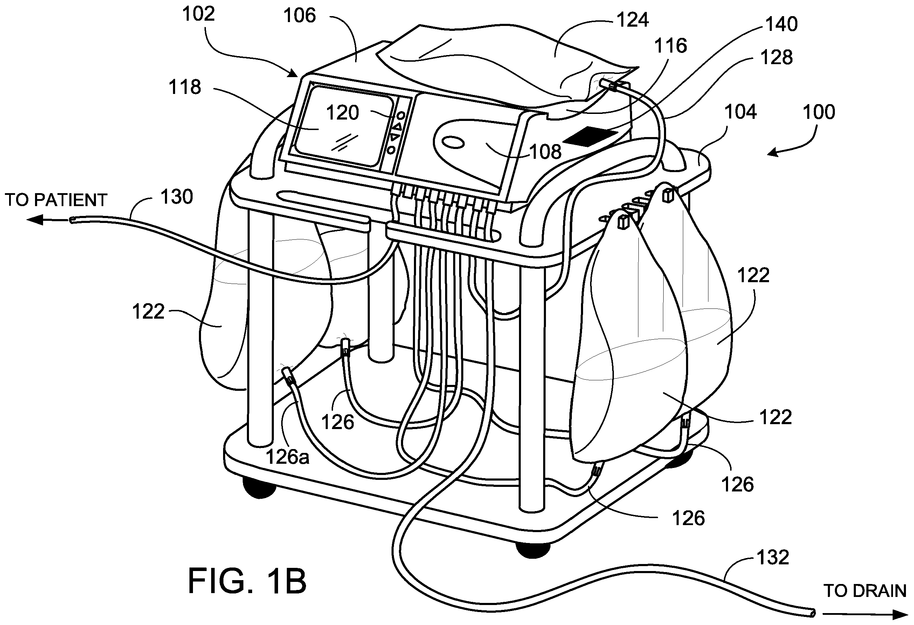

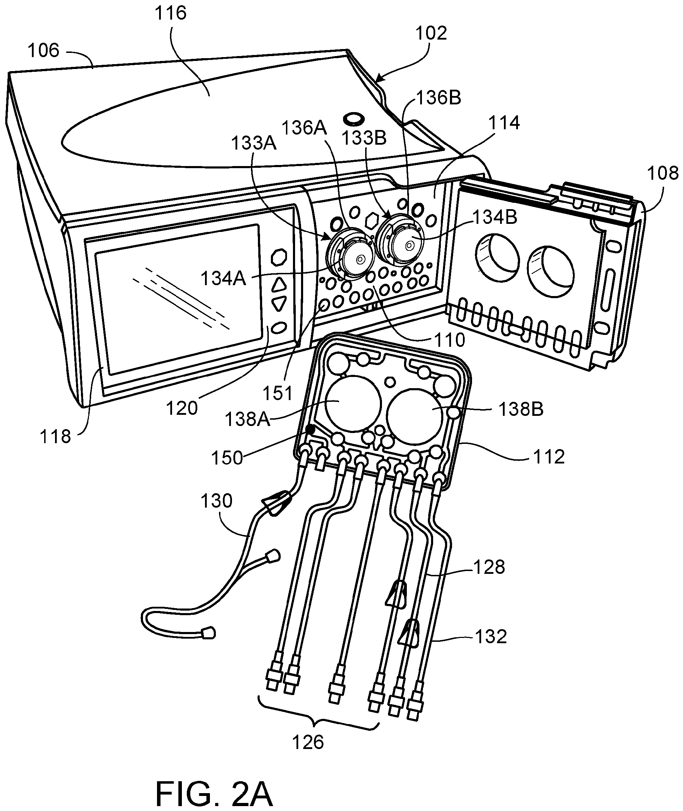

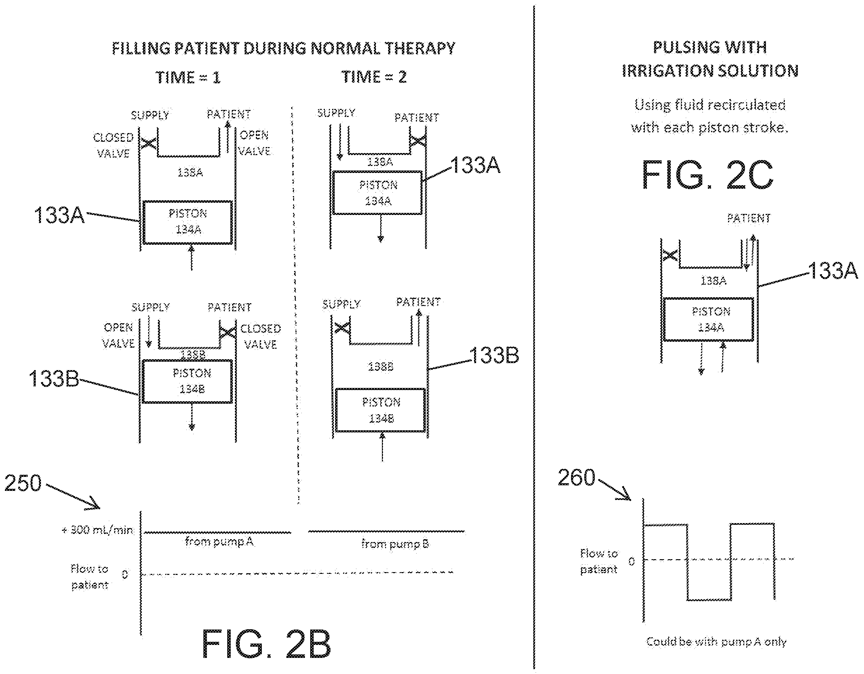

FIG. 1A is a perspective view of a peritoneal dialysis ("PD") system 100 that is attached to a patient 101 to provide a pulsed flow depicted by flow graph 260 as described further herein. FIG. 1B is a perspective view of the peritoneal dialysis ("PD") system 100. The PD system 100 includes a PD cycler 102 configured for attachment to a patient. The PD cycler 102 is positioned atop a portable cart 104. The PD cycler 102 is configured to pump medical fluid, such as dialysate, from the dialysis solutions bags 122 to a patient via a fluid exchange conduit, namely patient line 130. Pumping operations of the PD cycler 102 are controlled via controller 140. As described further herein, the PD cycler 102 includes a cassette 112 (shown in FIG. 2) positioned behind a door 108 of the PD cycler 102 and inside of a housing 106 of the PD cycler 102. The cassette 112 may include a disposable cassette. The patient line 130 can be connected to a patient's abdomen via a catheter and can be used to pass dialysis solution back and forth between the cassette 112 and the patient during treatment. The dialysis solution bags 122 are suspended from fingers on the sides of the cart 104. The dialysis solution bags 122 are connected to the cassette 112 via dialysis solution bag lines 126. The dialysis solution bag lines 126 can be used to pass dialysis solution from dialysis solution bags 122 to the cassette 112 during use, and the heater bag line 128 can be used to pass dialysis solution back and forth between the cassette 112 and the heater bag 124 during use. The heater bag 124 is positioned on a heater tray 116. The heater tray 116 is sized and shaped to accommodate a bag of dialysis solution (e.g., a 5 liter bag of dialysis solution). The patient line 130 can also be used to pass irrigation fluid contained in an irrigation fluid bag 122a to the patient line 130 via irrigation fluid bag line 126a and cassette 112. The irrigation fluid can be heated in the same manner as the dialysis solution. A drain line 132 can be connected to a drain or drain receptacle and can be used to pass spent dialysis solution or irrigation fluid drained from the patient to the cassette 112 and out to the drain or drain receptacle, for example after a dwell cycle. The PD cycler 102 also includes a touch screen 118 and additional control buttons 120 that can be operated by a user (e.g., a patient) to allow, for example, set-up, initiation, and/or termination of a PD treatment.

The PD cycler 102 may also include a data storage and/or transmission component that may be coupled to the control unit 140 and that may enable the storage of data on the PD machine and/or the transmission of data to and from the PD cycler 102. In various implementations, the data may include prescription information, treatment data and/or other therapy-based data and/or may include control or authorization information and/or other user profile-based information. In various implementations, the component 142 may include: a wired connection to a network/Internet, an interface for receiving a physical storage unit, e.g. a universal serial bus (USB) memory unit that may be used to transfer and receive data, and/or wireless transmission components for transmitting or receiving data and/or other signals wirelessly. The wireless transmission components may include components for short-range wireless transmission technologies and/or near field communications (NFC technologies), for communication with one or more peripheral devices in proximity to the PD cycler 102. Additionally or alternatively, the wireless transmission components may include network transmission components for transmitting and receiving data and/or other signals wirelessly via a telecommunications network and/or the Internet with one or more remote servers. In connection with transmission, data may be secured and/or encrypted/decrypted via the control unit 140 using appropriate security and encryption protocols according to applicable laws and regulations governing transmission of sensitive data and/or protected medical information.

Referring also to FIG. 2A, the PD cycler 102 includes a cassette interface 110 that abuts a disposable PD cassette 112 when the cassette 112 is disposed within a cassette compartment 114 formed between the cassette interface 110 and the closed door 108. The PD cycler 102 includes pistons 133A, 133B with piston heads 134A, 134B attached to piston shafts that can be axially moved within piston access ports 136A, 136B formed in the cassette interface 110. The piston shafts are connected to motors that can be operated to move the piston heads 134A, 134B axially inward and outward within the piston access ports 136A, 136B. When the cassette 112 is positioned within the cassette compartment 114 of the PD cycler 102 with the door 108 closed, the piston heads 134A, 134B of the PD cycler 102 align with pump chambers 138A, 138B of the cassette 112 such that the piston heads 134A, 134B can be mechanically connected to fastening members of the cassette 112 overlying the pump chambers 138A, 138B. As a result of this arrangement, movement of the piston heads 134A, 134B toward the cassette 112 during treatment can decrease the volume of the pump chambers 138A, 138B, and force dialysis solution out of the pump chambers 138A, 138B, while retraction of the piston heads 134A, 134B away from the cassette 112 can increase the volume of the pump chambers 138A, 138B and cause dialysis solution to be drawn into the pump chambers 138A, 138B.

As will be described in greater detail below in connection with FIGS. 2B and 2C, the piston heads 134A, 134B can be operated in a manner during a PD treatment to generate fluid pulses that pass through the cassette 112, the patient line 130, and the PD catheter and into the patient's peritoneal cavity. It is believed that such fluid pulses can cause motion in the catheter that loosens the biofilm from the catheter and thus decrease the risk of peritonitis. It is also believed that such fluid pulses can create a mixing of fluid in a patient cavity to reach and mix with stagnant pockets of infection.

FIGS. 2B and 2C are schematics of operation of piston pumps of the PD cycler of FIG. 2A. FIG. 2B provides a schematic illustration of the operation of pistons 133A and 133B during normal operation. The PD cycler 102 operates to generate a continuous flow to the patient. In particular, during Time 1 piston 133A pumps to the patient via a valve open to the patient line while the supply valve that supplies fluid for pumping by the piston 133A is closed. As the piston 133A pumps to the patient, the piston 133B is drawing fluid from the supply bag. The cycle of the pistons alternate at Time 2, as the piston 133A runs inversely with the piston 133B, 180 degrees out of phase with one another. At Time 2, the piston 133B pumps to the patient via a valve open to the patient line while the supply valve that supplies fluid for pumping by the piston 133B is closed. As shown by the graph 250, a steady flow to the patient is produced by the out of phase operation of the pumps of 133A and 133B, to pump fluid from a supply source to the patient.

FIG. 2C is a schematic illustrating a pulsatile flow scheme for pumping irrigation fluid to the patient. The pulsatile flow is provided here by a single piston 133A or 133B, but can be produced using multiple pistons, both 133A and 133B. The piston 133A pumps irrigation fluid to and from the patient line, recirculating the fluid back and forth to and from the patient line with each piston stroke. The piston 133A, thereby produces a flow depicted by flow graph 260, rather than a continuous flow.

While fluid pulses may be generated directly by the piston heads 134A, 134B of the PD cycler 102 in some implementations, other implementations may involve the use of additional components to create certain fluid pulses. For example, in particular implementations the cassette 112 includes a valve 150 configured to open and close a fluid communication pathway between the pump chambers 138A, 138B of the cassette 112 and the patient line 130. The valve 150 is configured for coupling to an actuator 151 for actuating the valve 150 in response to control signals from the controller 140. The valve 150 may include a rotary valve such as a ball valve. The ball valve may have a tapered opening that decreases in cross sectional area across a diameter of the ball valve, thereby forming a nozzle. The tapered opening causes fluid flowing through the valve to experience and increase in velocity of the fluid released by the valve 150. The valve 150 may be closed (e.g. by actuation of the actuator 151 via controller 140) in concert with actuation of the piston heads 134A, 134B in order to increase the fluid pressure of medical fluid contained in the pump chambers 138A, 138B over a plurality of strokes of the piston heads 134A, 134B or other pumping actuators. The valve 150 may be opened intermittently to release a fluid pulse of the medical fluid built up behind the closed valve 150. The valve 150 is opened under the control of controller 140 to release the fluid pressure pulse into the patient line 130 and is then closed again to build up a new fluid pulse.

In alternative implementations, the actuator 151 of the PD cycler 102 includes an inflatable valve configured to build up pressure in the cassette 112 for a fluid pulse. The inflatable valve is connected to a valve manifold that applies a vacuum pressure or positive air to the inflatable valve to hold the inflatable valve in an inflated or a deflated state. In the inflated state, the inflatable valve blocks fluid flow through a passage in the cassette 112 (for example by filling or causing a membrane of cassette 112 to fill a dome region 153 of cassette 112) and in a deflated state, the inflatable valve permits fluid to flow through the passage. Inflation and deflation of the valve can be controlled by the controller 140 to allow a fluid pressure pulse to be generated in the cassette 112 and injected from the cassette 112 into the patient line 130.

The pulsing frequency provided by the PD cycler 102 to pulse irrigation fluid to the patient cavity via the patient line can be regulated by altering actuation of the pistons alone or the valves alone. For example, in certain embodiments the opening frequency of the valve 150 may be specified for a slow pulse, such as pulses injected at 1Hz or less and may be specified for a fast pulse, such as pulses injected in the 100Hz to 500Hz range.

In certain embodiments, the pulse frequency may be controlled by the controller 140 causing the piston heads 134A, 134B to be operated at a particular frequency, for example at a higher frequency than the frequency of operation of the piston heads 134A, 134B during a normal continuous pumping cycle used to fill the patient cavity. The higher operating frequency of the piston heads 134A, 134B is implemented in order to generate a prescribed fluid pulse frequency in the patient line 130 upon opening the valve 150.

While the dialysis system has been principally described herein as including a piston based pump system, as discussed further herein, certain embodiments may be implemented with an alternative pumping system including, but not limited to, a piezoelectric pump system. For example, piezoelectric components, membranes, or strips may be housed within the housing 106 of the PD cycler 102 (e.g. in place of piston heads 134A, 134B) and may be activated and deactivated to expand and contract the pump chambers 138A, 138B of the cassette 112 to force the medical fluid solution into and/or out of the cassette 112. Certain implementations may include a piezo-electric pump configured as described in U.S. Patent No. 8,267,885.

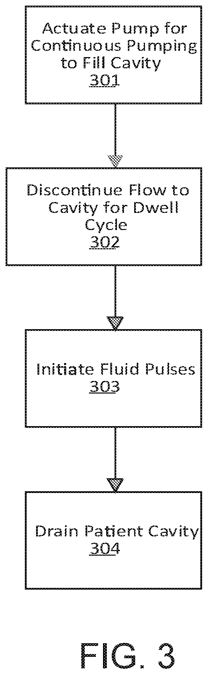

FIG. 3 is a flow diagram demonstrating operation of a PD system 100 for pulsing medical fluid through a patient line 130 to a catheter and a patient cavity. In particular, the PD cycler 102 includes the controller 140 housed within housing 106 and communicably coupled to the motors actuating the piston heads 134A, 134B. In contrast to the continuous operation of the pump exhibited during a normal pump cycle to fill a patient cavity with medical fluid, such as dialysate, from the dialysis solutions bags 122, the controller 140 is configured to actuate the piston heads 134A, 134B to cause the PD cycler 102 to transmit fluid pulses to the patient line 130. In certain implementations, the controller 140 controls actuation of the valve 150 in fluid communication with the patient line 130 to open and close in concert with actuation of the piston heads 134A, 134B. The controller 140 may be specifically configured to cause the PD cycler 102 to transmit fluid pulses to the patient line 130 during a dwell cycle, as discussed further herein.

As demonstrated in FIG. 3, the controller 140 activates the PD cycler 102 at 301 to pump a medical fluid from a medical fluid pump chamber, such as chambers 138A, 138B of cassette 112, and through a patient line 130 to a cavity of the patient. The controller 140 may activate the PD cycler 102 for a particular duration to pump a prescribed volume of fluid, such as dialysate or irrigation fluid to a patient cavity, such as the peritoneum or peritoneal cavity. At 302, the PD cycler 102 discontinues pumping from the cassette 112 to the patient line 130 for a predetermined period of time. The period of time is generally referred to as the dwell period or dwell cycle. At 303, the controller 140 causes the PD cycler 102 to initiate pulsed fluid flow from the cassette 112 to the patient line 130. The pulsed fluid flow, in contrast to the continuous flow, includes a series of fluid pulses to and from the patient line 130, or a series of high pressure fluid pulses injected into the patient line 130 intermittently. The fluid pulses are repeatedly built up to a prescribed high pressure and released by the PD cycler 102. The pulsed fluid flow causes motion to the dwelling fluid in the catheter and patient cavity. In certain implementations, the pulsed fluid may include pulsed irrigation fluid from irrigation fluid bag 122a and the pulsed irrigation fluid may be recirculated with each piston stroke (e.g. by draining to the cassette 112 for recirculation to the patient cavity). In particular implementations, the pulsed fluid includes medical fluid from multiple piston strokes. The pulsed fluid may be at particular frequencies, which frequencies may include frequencies corresponding to determined resonant frequencies of a catheter connecting the patient cavity to the patient line 130. After one or more fluid pulses, the controller 140 causes the PD cycler 102 to execute a drain cycle at 304 to remove the fluid from the patient cavity.

As discussed herein, the patient cavity may be connected to the patient line 130 via a catheter, such as a tenckhoff catheter. The controller 140 may control the PD cycler 102 to generate fluid pulses to cause the catheter to expand and contract at particular locations corresponding to the resonant mode shape(s) of the catheter so as to cause biofilm to be removed.

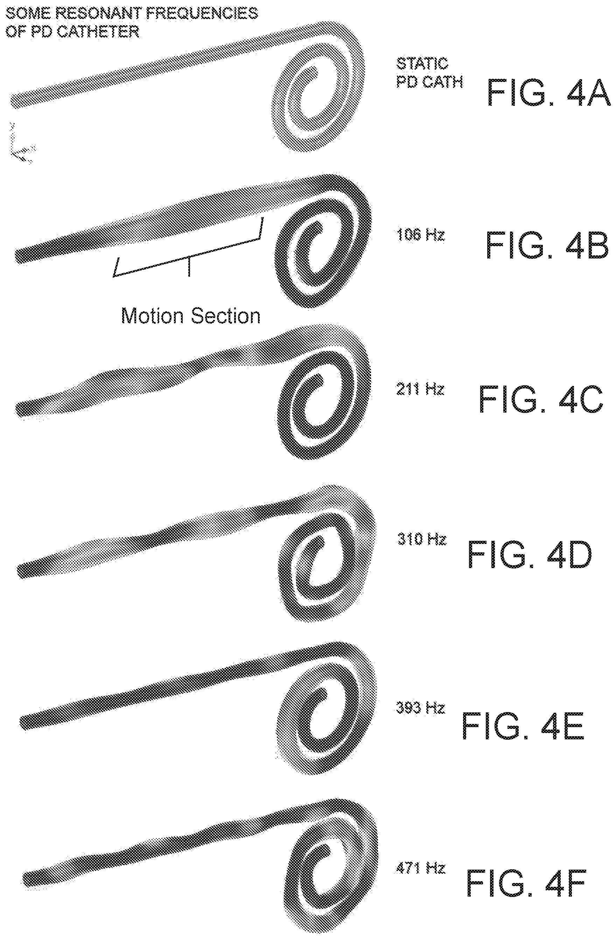

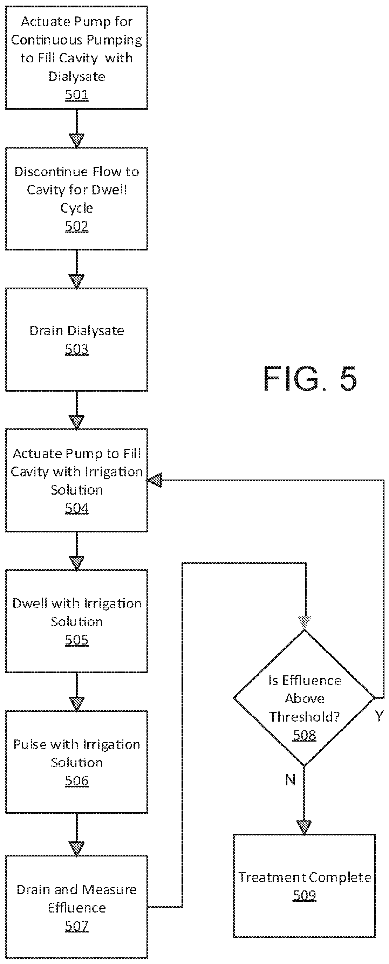

FIGS. 4A-4F illustrate by way of example finite element analysis (FEA) used to determine resonant frequencies of a tenckhoff catheter. The illustrated FEA model is based on a silicone catheter having a young's modulus = 5e6 Pascal, a density of 1,250 kg/m³, and Poisson's ratio of 0.4. FIG. 4A demonstrates a static catheter. FIG. 4B illustrates a catheter pulsed at a fluid pressure of 106 Hz causing motion in section 401, a straight section of the catheter. As demonstrated in FIGS. 4C-4F, pulsing the fluid at other frequencies, identified in each figure, can produce motion in other sections of the catheter beyond the straight section. FIGS. 4B-4F illustrate by way of non-limiting example, various resonant modes of a catheter excited by fluid pulses in the range of 100-500 Hz. In certain implementations, the controller 140 may implement a sinusoidal sweep of fluid pulses to generate motions that cause regions of excitation that overlap those shown in FIGS. 4B-4F along the catheter. For example, a sinusoidal sweep could be initiated by generating fluid pulses at a frequency of 100 Hz and the frequency of the fluid pressure pulses may be increased incrementally until the fluid pulse frequency reaches 500 Hz. In certain implementations, the fluid pulses may be generated at a plurality of random frequencies that encompasses 100 Hz to 500 Hz. Accordingly by resonating the catheter over a wide range of frequencies, either with a sinusoidal sweep from low to high frequencies (such as 100 Hz to 500 Hz) or with a random vibration that excites all of these frequencies simultaneously, the vibratory motion of the entire catheter loosens biofilm anywhere on the catheter. FIG. 5 is a flow diagram demonstrating operation of the PD system 100 for pulsing the fluid pressure responsive to an optical sensor positioned along the fluid path of the fluid from the cassette 112 to the patient cavity and back. The optical sensor system may be implemented as described in U.S. Patent Nos. 8,728,023, 8,777,891, and/or 8,801,652. At 501, the pump is activated to cause pumping of the medical fluid, such as dialysate, from the cassette 112 to the patient line 130. At 502 flow to the cavity from the pump is discontinued to permit a dwell cycle to occur. During this dwell period, toxins cross the peritoneum of the patient into the dialysis solution from the patient's blood. An irrigation cycle may be instituted in combination with the PD cycle as demonstrated in FIG. 5. Accordingly, at 503 the dialysate is drained from the patient cavity. At 504, the pump is actuated to fill the cavity with an irrigation fluid pumped from an irrigation bag, through the cassette 112 to the patient via patient line 130. In certain implementations, the irrigation fluid may be heated before being pumped to the cavity. The irrigation fluid may be heated to a temperature substantially higher than room temperature. For example, the irrigation fluid may be heated to a temperature substantially above room temperature, such as normal body temperature (98.6 degrees Fahrenheit or 37 degrees Celsius). It is believed that the warmed irrigation fluid increases the molecular motion in the fluid to help reduce adherence of bacteria to the patient cavity or the catheter during the irrigation treatment. At 505, the irrigation fluid is permitted to dwell in the patient cavity. At 506 the pump is used to pulse the irrigation fluid. At 507, the irrigation fluid is drained from the patient cavity and the effluence of the irrigation fluid is measured. At 508, an analysis is done to compare the effluence measurement to a predetermined threshold. If the effluence measurement indicates that the effluence measurement is below the threshold, indicating that a bacterial infection in the cavity is unlikely, the controller 140 continues to drain the patient cavity at 509. If the effluence measurement indicates that the effluence measurement is above a predetermined threshold, indicating high likelihood of bacterial infection in the cavity, the controller actuates the pump again at 504 to re-fill the cavity with irrigation fluid.

In particular embodiments, the controller 140 receives an input from an optical sensor measuring the effluent white blood cell count of medical fluid flowing back from the patient to the cassette 112 or a designated drain. The difference in the effluence measurement and the threshold may be determined and may be used to determine pulsing parameters including, but not limited to, pulse frequency and duration of pulsing. The irrigation cycles can be repeated until the effluent white blood cell count is within a predetermined identified percentage of the patient's infection free effluent white blood cell count. The irrigation cycles can be repeated until the effluent white blood cell count is reduced by a predetermined level. The PD cycler 102 may also be pre-programmed to time-out after a specific number of irrigation cycles has been reached.

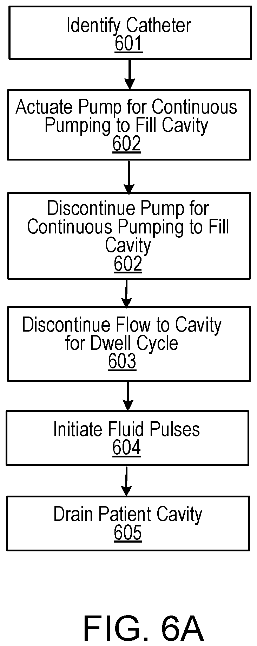

FIG. 6A is a flow diagram demonstrating operation of the PD system 100 for pulsing the fluid pressure of a catheter coupled to the PD system. At 601, the catheter connected to the PD cycler 102 of the PD system 100 is identified. The catheter may be identified by type and the PD cycler may have a preprogrammed memory storing information related to the material properties of the catheter, including but not limited to length, elasticity, density, or other dimensional or material properties. These material properties may be used to determine resonant modes of the catheter. At 602, the pump is activated to cause pumping of medical fluid from a medical fluid bag through the cassette 112 and to the patient line 130. At 603, the pumped medical fluid is permitted to dwell in the patient cavity for a predetermined period of time, depending on the treatment, fluid, and patient. At 604, fluid pulses are initiated via the pump. At 605, the patient cavity is drained.

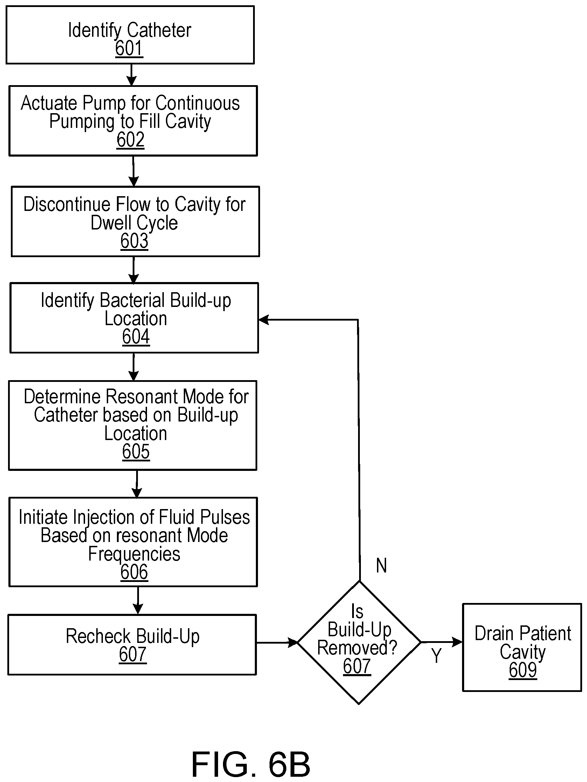

FIG. 6B is a flow diagram demonstrating operation of the PD system 100 for pulsing the fluid pressure of a catheter coupled to the PD system, with an imaging device of one or more sensors positioned along the catheter to detect cross-sectional changes of the catheter along a length of the catheter to identify a location of bacterial buildup. In certain implementations, the location of the buildup may be manually identified and entered into the PD cycler 102 via the user interface, the touch screen 118. In such an embodiment, the controller 140 can be used to determine the appropriate resonant frequency or frequencies of the catheter required to disturb the bacterial buildup based on the identification of the location(s) of the bacterial buildup on the catheter. As a result of the one or more resonant frequencies identified, the controller 140 causes the PD cycler 102 to pulse the medical fluid in the catheter via the patient line 130 at the identified frequency or frequencies. The pulsed medical fluid may be an irrigation fluid injected into the catheter via patient line 130 during the dwell cycle. At 607, the catheter is rechecked for bacterial build-up, in a manner corresponding to that discussed in connection with the process at 604. In connection with rechecking for bacterial build up, an analysis of the results is made at 608 and a decision is made as to whether or not further fluid pulsing is required. If analysis 608 determines that the build-up has been successfully removed, the medical fluid is drained at 609 via the PD cycler 102. If the analysis at 608 determines that the build-up is still present, pulsing may be repeated at 606.

While the dialysis system has been principally described herein as being a peritoneal dialysis system, other medical treatment systems can employ the techniques described herein. Examples of other medical treatments include treatment of urinary tract infections, bladder infection, or infection of other internal body cavities.

Implementations of the subject matter and the operations described in this specification can be implemented by digital electronic circuitry, or via computer software, firmware, or hardware, including the structures disclosed in this specification and their structural equivalents, or in combinations of one or more of them. Implementations of the subject matter described in this specification can be implemented as one or more computer programs, i.e., one or more modules of computer program instructions, encoded on computer storage medium for execution by, or to control the operation of, data processing apparatus.

A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

The operations described in this specification can be implemented as operations performed by a data processing apparatus on data stored on one or more computer-readable storage devices or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., a FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's user device in response to requests received from the web browser.

Implementations of the subject matter described in this specification can be implemented in a computing system that includes a back end component, e.g., as a data server, or that includes a middleware component, e.g., an application server, or that includes a front end component, e.g., a user computer having a graphical display or a Web browser through which a user can interact with an implementation of the subject matter described in this specification, or any combination of one or more such back end, middleware, or front end components. The components of the system can be interconnected by any form or medium of digital data communication, e.g., a communication network, and may provide for data and/or control information transfer over wireless short-range or near field communication networks to or from one or more nearby peripheral or other computing devices and/or over wireless long-distance telecommunication networks to or from one or more remote servers. Examples of communication networks include a local area network ("LAN") and a wide area network ("WAN"), an inter-network (e.g., the Internet), and peer-to-peer networks (e.g., ad hoc peer-to-peer networks).

The computing system can include users and servers. A user and server are generally remote from each other and typically interact through a communication network. The relationship of user and server arises by virtue of computer programs running on the respective computers and having a user-server relationship to each other. In some implementations, a server transmits data (e.g., an HTML page) to a user device (e.g., for purposes of displaying data to and receiving user input from a user interacting with the user device). Data generated at the user device (e.g., a result of the user interaction) can be received from the user device at the server.

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable sub combination. Moreover, although features may be described above as acting in certain combinations and even initially claimed as such, one or more features from a claimed combination can in some cases be excised from the combination, and the claimed combination may be directed to a sub combination or variation of a sub combination.

For the purpose of this disclosure, the term "coupled" means the joining of two members directly or indirectly to one another. Such joining may be stationary or moveable in nature. Such joining may be achieved with the two members or the two members and any additional intermediate members being integrally formed as a single unitary body with one another or with the two members or the two members and any additional intermediate members being attached to one another. Such joining may be permanent in nature or may be removable or releasable in nature.

It should be noted that the orientation of various elements may differ according to other exemplary implementations, and that such variations are intended to be encompassed by the present disclosure. It is recognized that features of the disclosed implementations can be incorporated into other disclosed implementations.

While various inventive implementations have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive implementations described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive implementations described herein. It is, therefore, to be understood that the foregoing implementations are presented by way of example only and that, within the scope of the appended claims and equivalents thereto, inventive implementations may be practiced otherwise than as specifically described and claimed. Inventive implementations of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

Also, the technology described herein may be embodied as a method, of which at least one example has been provided. The acts performed as part of the method may be ordered in any suitable way. Accordingly, implementations may be constructed in which acts are performed in an order different than illustrated, which may include performing some acts simultaneously, even though shown as sequential acts in illustrative implementations.

The claims should not be read as limited to the described order or elements unless stated to that effect.

## Claims

1. A medical fluid pumping system comprising:
a medical fluid pump chamber (138A, 138B);
a fluid exchange conduit (126, 128, 132) fluidly coupled to the medical fluid pump chamber;
at least one pump (133A, 133B, 134A, 134B) configured to pump medical fluid to and drain medical fluid from a cavity of a patient via a patient line (130) coupled to the fluid exchange conduit wherein the patient line includes a catheter and wherein the at least one pump is configured to pump dialysate to and drain dialysate from a peritoneal cavity of the patient via the patient line; and
a pump controller (140) communicably coupled to the at least one pump, the pump controller configured to cause the pump to transmit fluid pulses through the patient line, wherein the pump controller is configured to cause the pulses to occur at a resonant frequency of the catheter.

2. The medical fluid pumping system according to claim 1, wherein the pump controller is configured to cause the pump to transmit fluid pulses during a dwell cycle.

3. The medical fluid pumping system according to any one of the preceding claims, wherein the pump controller is configured to cause the pulses to occur at a rate in the range of 100Hz to 500Hz.

4. The medical fluid pumping system according to any one of the preceding claims, wherein the pump controller is configured to cause the pulses to occur sinusoidally.

5. The medical fluid pumping system according to any one of the preceding claims, wherein the pump controller is configured to cause the at least one pump to pump dialysate into the peritoneal cavity before causing the at least one pump to transmit fluid pulses into the peritoneal cavity during a dwell cycle.

6. The medical fluid pumping system according to any one of the preceding claims, wherein the pump controller is configured to cause the at least one pump to drain fluid from the peritoneal cavity via the patient line after causing the at least one pump to transmit fluid pulses to the fluid compartment during the dwell cycle.

7. The medical fluid pumping system according to any one of the preceding claims, wherein the medical fluid pump chamber is defined by a disposable cassette.

8. The medical fluid pumping system according to any one of the preceding claims, further comprising an optical sensor coupled to at least one of the fluid exchange conduit and the medical fluid pump chamber, the optical sensor communicably coupled to the pump controller, and optionally wherein the pump controller is configured to cause the at least one pump to transmit fluid pulses in response to a reading of the optical sensor being above or below a predetermined threshold.

9. The medical fluid pumping system according to any one of the preceding claims, further comprising a first container (122) and a second container (122a) in fluid communication with the fluid exchange conduit, the first container containing the dialysate, the second container containing an irrigation fluid.

10. The medical fluid pumping system according to any one of the preceding claims, wherein the at least one pump includes either a piston pump or a piezoelectric pump.

11. A medical fluid pumping system comprising:
a medical fluid pump chamber (138A, 138B);
a fluid exchange conduit (126, 128, 132) fluidly coupled to the medical fluid pump chamber;
at least one pump (133A, 133B, 134A, 134B) configured to pump medical fluid to and drain medical fluid from a cavity of a patient via a patient line coupled to the fluid exchange conduit wherein the at least one pump is configured to pump dialysate to and drain dialysate from a peritoneal cavity of the patient via the patient line;
a rotary insert (150) positioned in the fluid exchange conduit;
an actuator coupled to the rotary insert; and
a flow controller electrically coupled to the actuator to cause the rotary insert to rotate in concert with actuation of the at least one pump to transmit fluid pulses to the medical fluid in the cavity during a dwell cycle, wherein the flow controller is configured to cause the fluid pulses to occur at a resonant frequency of the patient line.

12. The medical fluid pumping system according to claim 11, wherein the flow controller is configured to cause the pulses to occur at a rate in the range of 100Hz to 500Hz.

13. The medical fluid pumping system according to any one of claims 11-12, wherein the flow controller is configured to cause the pulses to occur sinusoidally.

14. The medical fluid pumping system according to any one of claims 11-13, wherein the medical fluid pump chamber is defined by a disposable cassette.

15. The medical fluid pumping system according to any one of claims 11-14, further comprising an optical sensor coupled to at least one of the fluid exchange conduit and the medical fluid pump chamber, the optical sensor communicably coupled to the flow controller, and optionally wherein the flow controller is configured to cause transmission of fluid pulses in response to a reading of the optical sensor being above or below a predetermined threshold.

## Patentansprüche

1. Medizinisches Flüssigkeitspumpsystem, umfassend:
eine medizinische Flüssigkeitspumpkammer (138A, 138B);
eine Flüssigkeitsaustauschrohrleitung (126, 128, 132), die fließtechnisch an die medizinische Flüssigkeitspumpkammer gekoppelt ist;
mindestens eine Pumpe (133A, 133B, 134A, 134B), die ausgestaltet ist, um medizinische Flüssigkeit mittels einer Patientenleitung (130), die an die Flüssigkeitsaustauschrohrleitung gekoppelt ist, in einen Hohlraum eines Patienten zu pumpen und medizinische Flüssigkeit daraus abzuziehen, wobei die Patientenleitung einen Katheter einschließt, und wobei die mindestens eine Pumpe ausgestaltet ist, um mittels der Patientenleitung Dialysat zu der Bauchhöhle des Patienten zu pumpen und Dialysat aus der Bauchhöhle abzuziehen; und
eine Pumpensteuerung (140), die kommunikativ an die mindestens eine Pumpe gekoppelt ist, wobei die Pumpensteuerung ausgestaltet ist, um zu bewirken, dass die Pumpe Flüssigkeitsimpulse durch die Patientenleitung hindurch übermittelt, wobei die Pumpensteuerung ausgestaltet ist, um zu bewirken, dass die Impulse bei einer Resonanzfrequenz des Katheters stattfinden.

2. Medizinisches Flüssigkeitspumpsystem nach Anspruch 1, wobei die Pumpensteuerung ausgestaltet ist, um zu bewirken, dass die Pumpe während eines Verweilzyklus Flüssigkeitsimpulse übermittelt.

3. Medizinisches Flüssigkeitspumpsystem nach einem der vorhergehenden Ansprüche, wobei die Pumpensteuerung ausgestaltet ist, um zu bewirken, dass die Impulse mit einer Rate im Bereich von 100 Hz bis 500 Hz stattfinden.

4. Medizinisches Flüssigkeitspumpsystem nach einem der vorhergehenden Ansprüche, wobei die Pumpensteuerung ausgestaltet ist, um zu bewirken, dass die Impulse sinusförmig stattfinden.

5. Medizinisches Flüssigkeitspumpsystem nach einem der vorhergehenden Ansprüche, wobei die Pumpensteuerung ausgestaltet ist, um zu bewirken, dass die mindestens eine Pumpe Dialysat in die Bauchhöhle pumpt, bevor bewirkt wird, dass die mindestens eine Pumpe während des Verweilzyklus Flüssigkeitsimpulse in die Bauchhöhle übermittelt.

6. Medizinisches Flüssigkeitspumpsystem nach einem der vorhergehenden Ansprüche, wobei die Pumpensteuerung ausgestaltet ist, um zu bewirken, dass die mindestens eine Pumpe Flüssigkeit aus der Bauchhöhle mittels der Patientenleitung abzieht, nachdem bewirkt worden ist, dass die mindestens eine Pumpe während des Verweilzyklus Flüssigkeitsimpulse in die Bauchhöhle übermittelt.

7. Medizinisches Flüssigkeitspumpsystem nach einem der vorhergehenden Ansprüche, wobei die medizinische Flüssigkeitspumpkammer durch eine Einwegkassette definiert ist.

8. Medizinisches Flüssigkeitspumpsystem nach einem der vorhergehenden Ansprüche, das des Weiteren einen optischen Sensor umfasst, der an mindestens eine von der Flüssigkeitsaustauschrohrleitung und der medizinischen Flüssigkeitspumpkammer gekoppelt ist, wobei der optische Sensor kommunikativ an die Pumpensteuerung gekoppelt ist, und wobei die Pumpensteuerung gegebenenfalls ausgestaltet ist, um zu bewirken, dass die mindestens eine Pumpe Flüssigkeitsimpulse in Reaktion darauf übermittelt, dass eine Ablesung des optischen Sensors oberhalb oder unterhalb eines vorgegebenen Schwellenwerts liegt.

9. Medizinisches Flüssigkeitspumpsystem nach einem der vorhergehenden Ansprüche, des Weiteren umfassend einen ersten Behälter (122) und einen zweiten Behälter (122a) in Fließkommunikation mit der Flüssigkeitsaustauschrohrleitung, wobei der erste Behälter das Dialysat enthält und der zweite Behälter eine Spülflüssigkeit enthält.

10. Medizinisches Flüssigkeitspumpsystem nach einem der vorhergehenden Ansprüche, wobei die mindestens eine Pumpe entweder eine Kolbenpumpe oder eine piezoelektrische Pumpe einschließt.

11. Medizinisches Flüssigkeitspumpsystem, umfassend:
eine medizinische Flüssigkeitspumpkammer (138A, 138B);
eine Flüssigkeitsaustauschrohrleitung (126, 128, 132), die fließtechnisch an die medizinische Flüssigkeitspumpkammer gekoppelt ist;
mindestens eine Pumpe (133A, 133B, 134A, 134B), die ausgestaltet ist, um medizinische Flüssigkeit mittels einer Patientenleitung, die an die Flüssigkeitsaustauschrohrleitung gekoppelt ist, in einen Hohlraum eines Patienten zu pumpen und medizinische Flüssigkeit daraus abzuziehen, wobei die mindestens eine Pumpe ausgestaltet ist, um mittels der Patientenleitung Dialysat zu der Bauchhöhle des Patienten zu pumpen und Dialysat aus der Bauchhöhle abzuziehen;
einen Dreheinsatz (150), der in der Flüssigkeitsaustauschrohrleitung positioniert ist; einen Aktor, der an den Dreheinsatz gekoppelt ist; und
eine Durchflusssteuerung, die elektrisch an den Aktor gekoppelt ist, um zu bewirken, dass der Dreheinsatz in Übereinstimmung mit der Betätigung der mindestens einen Pumpe gedreht wird, um während eines Verweilzyklus Flüssigkeitsimpulse an die medizinische Flüssigkeit in dem Hohlraum zu übermitteln, wobei die Durchflusssteuerung ausgestaltet ist, um zu bewirken, dass die Flüssigkeitsimpulse an einer Resonanzfrequenz der Patientenleitung stattfinden.

12. Medizinisches Flüssigkeitspumpsystem nach Anspruch 11, wobei die Durchflusssteuerung ausgestaltet ist, um zu bewirken, dass die Impulse mit einer Rate im Bereich von 100 Hz bis 500 Hz stattfinden.

13. Medizinisches Flüssigkeitspumpsystem nach einem der Ansprüche 11 bis 12, wobei die Durchflusssteuerung ausgestaltet ist, um zu bewirken, dass die Impulse sinusförmig stattfinden.

14. Medizinisches Flüssigkeitspumpsystem nach einem der Ansprüche 11 bis 13, wobei die medizinische Flüssigkeitspumpkammer durch eine Einwegkassette definiert ist.

15. Medizinisches Flüssigkeitspumpsystem nach einem der Ansprüche 11 bis 14, das des Weiteren einen optischen Sensor umfasst, der an mindestens eine von der Flüssigkeitsaustauschrohrleitung und der medizinischen Flüssigkeitspumpkammer gekoppelt ist, wobei der optische Sensor kommunikativ an die Durchflusssteuerung gekoppelt ist, und wobei die Durchflusssteuerung gegebenenfalls ausgestaltet ist, um die Übermittlung von Flüssigkeitsimpulsen in Reaktion darauf zu bewirken, dass eine Ablesung des optischen Sensors oberhalb oder unterhalb eines vorgegebenen Schwellenwerts liegt.

## Revendications

1. Système de pompage de fluide médical comprenant :
une chambre de pompe à fluide médical (138A, 138B) ;
une conduite d'échange de fluide (126, 128, 132) couplée fluidiquement à la chambre de pompe à fluide médical ;
au moins une pompe (133A, 133B, 134A, 134B) configurée pour pomper un fluide médical jusqu'à et drainer un fluide médical depuis une cavité d'un patient par le biais d'une ligne de patient (130) couplée à la conduite d'échange de fluide, la ligne de patient comportant un cathéter et l'au moins une pompe étant configurée pour pomper un dialysat jusqu'à et drainer un dialysat depuis une cavité péritonéale du patient par le biais de la ligne de patient ; et
un contrôleur de pompe (140) couplé de façon communicante à l'au moins une pompe, le contrôleur de pompe étant configuré pour conduire la pompe à transmettre des impulsions de fluide à travers la ligne de patient, le contrôleur de pompe étant configuré pour faire en sorte que les impulsions se produisent à une fréquence de résonance du cathéter.

2. Système de pompage de fluide médical selon la revendication 1, dans lequel le contrôleur de pompe est configuré pour conduire la pompe à transmettre des impulsions de fluide pendant un cycle de maintien.

3. Système de pompage de fluide médical selon l'une quelconque des revendications précédentes, dans lequel le contrôleur de pompe est configuré pour faire en sorte que les impulsions se produisent à une fréquence dans la gamme de 100 Hz à 500 Hz.

4. Système de pompage de fluide médical selon l'une quelconque des revendications précédentes, dans lequel le contrôleur de pompe est configuré pour faire en sorte que les impulsions se produisent sinusoïdalement.

5. Système de pompage de fluide médical selon l'une quelconque des revendications précédentes, dans lequel le contrôleur de pompe est configuré pour conduire l'au moins une pompe à pomper un dialysat à l'intérieur de la cavité péritonéale avant de conduire l'au moins une pompe à transmettre des impulsions de fluide à l'intérieur de la cavité péritonéale pendant un cycle de maintien.

6. Système de pompage de fluide médical selon l'une quelconque des revendications précédentes, dans lequel le contrôleur de pompe est configuré pour conduire l'au moins une pompe à drainer un fluide depuis la cavité péritonéale par le biais de la ligne de patient après avoir conduit l'au moins une pompe à transmettre des impulsions de fluide au compartiment à fluide pendant le cycle de maintien.

7. Système de pompage de fluide médical selon l'une quelconque des revendications précédentes, dans lequel la chambre de pompe à fluide médical est définie par une cassette jetable.

8. Système de pompage de fluide médical selon l'une quelconque des revendications précédentes, comprenant en outre un capteur optique couplé à la conduite d'échange de fluide et/ou la chambre de pompe à fluide médical, le capteur optique étant couplé de façon communicante au contrôleur de pompe, et le contrôleur de pompe étant éventuellement configuré pour conduire l'au moins une pompe à transmettre des impulsions de fluide en réponse à une lecture du capteur optique qui est supérieure ou inférieure à un seuil prédéterminé.

9. Système de pompage de fluide médical selon l'une quelconque des revendications précédentes, comprenant en outre un premier récipient (122) et un deuxième récipient (122a) en communication fluidique avec la conduite d'échange de fluide, le premier récipient contenant le dialysat, le deuxième récipient contenant un fluide d'irrigation.

10. Système de pompage de fluide médical selon l'une quelconque des revendications précédentes, dans lequel l'au moins une pompe comporte une pompe à piston ou une pompe piézoélectrique.

11. Système de pompage de fluide médical comprenant :
une chambre de pompe à fluide médical (138A, 138B) ;
une conduite d'échange de fluide (126, 128, 132) couplée fluidiquement à la chambre de pompe à fluide médical ;
au moins une pompe (133A, 133B, 134A, 134B) configurée pour pomper un fluide médical jusqu'à et drainer un fluide médical depuis une cavité d'un patient par le biais d'une ligne de patient couplée à la conduite d'échange de fluide, l'au moins une pompe étant configurée pour pomper un dialysat jusqu'à et drainer un dialysat depuis une cavité péritonéale du patient par le biais de la ligne de patient ;
un insert rotatif (150) positionné dans la conduite d'échange de fluide ;
un actionneur couplé à l'insert rotatif ; et
un contrôleur d'écoulement couplé électriquement à l'actionneur pour faire tourner l'insert rotatif de concert avec l'actionnement de l'au moins une pompe pour transmettre des impulsions de fluide au fluide médical dans la cavité pendant un cycle de maintien, le contrôleur d'écoulement étant configuré pour faire en sorte que les impulsions de fluide se produisent à une fréquence de résonance de la ligne de patient.

12. Système de pompage de fluide médical selon la revendication 11, dans lequel le contrôleur d'écoulement est configuré pour faire en sorte que les impulsions se produisent à une fréquence dans la gamme de 100 Hz à 500 Hz.

13. Système de pompage de fluide médical selon l'une quelconque des revendications 11 et 12, dans lequel le contrôleur d'écoulement est configuré pour faire en sorte que les impulsions se produisent sinusoïdalement.

14. Système de pompage de fluide médical selon l'une quelconque des revendications 11 à 13, dans lequel la chambre de pompe à fluide médical est définie par une cassette jetable.

15. Système de pompage de fluide médical selon l'une quelconque des revendications 11 à 14, comprenant en outre un capteur optique couplé à la conduite d'échange de fluide et/ou la chambre de pompe à fluide médical, le capteur optique étant couplé de façon communicante au contrôleur d'écoulement, et le contrôleur d'écoulement étant éventuellement configuré pour induire la transmission d'impulsions de fluide en réponse à une lecture du capteur optique qui est supérieure ou inférieure à un seuil prédéterminé.
